# EUROPÄISCHE PATENTSCHRIFT

(11) **EP 0 666 254 B1**
(45) Veröffentlichungstag und Bekanntmachung des Hinweises auf die Patenterteilung: **14.04.1999**
(21) Anmeldenummer: 95101123.8
(22) Anmeldetag: 27.01.1995
(51) Int. Cl.: C07C 321/22, A01N 35/06, A01N 43/00

(54) **2-Aroylcydohexandione, Verfahren zu ihrer Herstellung sowie ihre Verwendung als Herbizide oder das Pflanzenwachstum regulierende Mittel**
2-Aroylcydohexanediones, process for their preparation and their use as herbicide or as plant growth regulating agent
2-Aroylcydohexanedoines, procédé pour leur préparation ainsi que leur utilisation comme herbicide ou comme agent régulateur de la croissance des plantes

(30) Priorität: 07.02.1994 DE 4403670
(43) Veröffentlichungstag der Anmeldung: 09.08.1995
(73) Patentinhaber: BASF AKTIENGESELLSCHAFT, 67056 Ludwigshafen (DE)
(72) Erfinder: Kast, Jürgen, Dr., D-67459 Böhl-Iggelheim (DE); von Deyn, Wolfgang, Dr., D-67434 Neustadt (DE); Nübling, Christoph, Dr., D-67454 Hassloch (DE); Walter, Helmut, Dr., D-67283 Obrigheim (DE); Gerber, Matthias, Dr., D-67117 Limburgerhof (DE); Westphalen, Karl-Otto, dr., D-67346 Speyer (DE)

(56) Entgegenhaltungen:
- EP-A- 0 243 313
- EP-A- 0 319 075
- EP-A- 0 487 454
- WO-A-93/16062

## Beschreibung

Die vorliegende Erfindung betrifft neue 2-Aroylcyclohexandione der Formel I 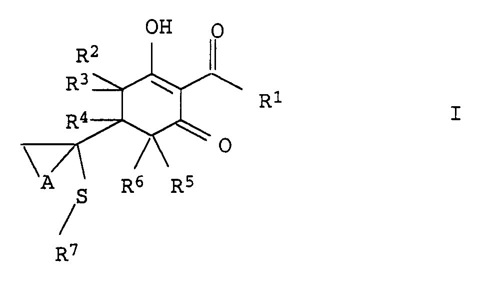 in der die Variablen folgende Bedeutung haben:
- A: Methylen;
- R¹: Phenyl, wobei Phenyl mit höchstens vier Substituenten substituiert sein kann, jeweils ausgewählt aus der Gruppe bestehend aus Halogen, Cyano, Nitro, -N=N-Ph, -S(O)ₘR⁸, (C₁-C₄-Alkoxy)carbonyl, -SO₂-N(R⁹)R¹⁰, -N(R⁹)-COR¹⁰, -N(R⁹)-SO₂R¹¹, C₁-C₄-Alkyl, C₁-C₄-Alkoxy, C₁-C₄-Halogenalkyl, C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano,
- Ph: steht für Phenyl, das unsubstituiert sein oder gewünschtenfalls ein bis drei Substituenten tragen kann, ausgewählt aus der Gruppe bestehend aus Halogen, Cyano, Nitro, -S(O)ₙR²², (C₁-C₄-Alkoxy)carbonyl, -SO₂-N(R²³)R²⁴, -N(R²³)-COR²⁴, -N(R²³)-SO₂R²⁵, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano;
- m,n: stehen jeweils für 0, 1 oder 2;
- R⁸, R²²: stehen unabhängig voneinander für C₁-C₄-Alkyl oder C₁-C₄-Halogenalkyl, wobei diese Gruppen ein oder zwei C₁-C₄-Alkoxy, C₁-C₄-Alkylthio- und/oder Cyanoreste tragen können;
- R⁹, R¹⁰, R²³, R²⁴: stehen unabhängig voneinander für Wasserstoff, C₁-C₄-Alkyl, C₁-C₄-Halogenalkyl, Phenyl oder Phenyl, das ein bis drei Reste trägt, ausgewählt aus der Gruppe Halgen, C₁-C₄-Alkyl und C₁-C₄-Alkoxy;
- R¹¹, R²⁵: stehen unabhängig voneinander für C₁-C₄-Alkyl oder C₁-C₄-Halogenalkyl, wobei diese Gruppen ein oder zwei Cyano-, Phenyl- und/oder Benzylreste tragen können;
- R², R³, R⁴, R⁵: unabhängig voneinander Wasserstoff oder C₁-C₄-Alkyl;
- R⁶: Wasserstoff, C₁-C₄-Alkyl oder (C₁-C₄-Alkoxy)carbonyl;
- R⁷: C₁-C₄-Alkyl;
mit der Maßgabe, daß mindestens ein Substituent am Phenyl einen Rest -S(O)ₘR⁸ mit m=2 und R⁸ C₁-C₄-Alkyl bedeutet,
sowie deren landwirtschaftlich brauchbaren Salze und die Ester von I mit C₁-C₁₀-Carbonsäuren oder anorganischen Säuren.

Außerdem betrifft die Erfindung Verfahren zur Herstellung dieser Verbindungen, ihre Verwendung als Herbizide und zur Regulierung des Pflanzenwachstums, herbizide Mittel und Mittel zur Regulierung des Pflanzenwachstums, welche diese Verbindungen als wirksame Substanzen enthalten, sowie Verfahren zur Herstellung dieser Mittel.

Des weiteren betrifft die Erfindung Verfahren zur Bekämpfung von unerwünschtem Pflanzenwuchs und Verfahren zur Regulierung des Pflanzenwachstums.

Herbizid wirksame 2-Aroylcyclohexandione sind bereits aus den folgenden Druckschriften bekannt: EP-A 90262, EP-A 135191, EP-A 186118, EP-A 186119, EP-A 186120, EP-A 319075, WO 90/05712, WO 91/01289, JP-A-03 052862 und JP-A-03 120202.

Des weiteren sind der EP-A 243 313 2-Aroylcyclohexandione vom Typ der Verbindungen I zu entnehmen, die in 5-Position einen 1-Alkylthiocycloalkylrest tragen.

Die herbiziden und das Wachstum der Pflanzen regulierenden Eigenschaften der bekannten Verbindungen können jedoch, besonders bei kleinen Aufwandmengen und -konzentrationen, nur bedingt befriedigen.

Aufgabe der vorliegenden Erfindung war es deshalb, weitere 2-Aroylcyclohexandione mit verbesserten Eigenschaften bereitzustellen.

Demgemäß wurden die eingangs definierten 2-Aroylcyclohexandione der Formel I gefunden. Weiterhin wurden Verfahren zur Herstellung dieser Verbindungen, ihre Verwendung als Herbizide oder zur Regulierung des Pflanzenwachstums, Herbizide und das Wachstum der Pflanzen regulierende Mittel, die die Verbindungen I enthalten, sowie Verfahren zur Bekämpfung von unerwünschtem Pflanzenwuchs und zur Regulierung des Pflanzenwuchses mit diesen Mitteln gefunden.

Die bei der Definition der Substituenten R¹ bis R²⁵ verwendeten Bedeutungen Halogen, C₁-C₄-Alkyl, (C₁-C₄-Alkyl)carbonyl, C₁-C₄-Alkoxy, (C₁-C₄-Alkoxy) carbonyl, C₁-C₄-Halogenalkyl und C₁-C₄-Halogenalkoxy stellen Sammelbegriffe für individuelle Aufzählungen der einzelnen Gruppenmitglieder dar. Die sechs letztgenannten Kohlenstoffketten können dabei geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom oder Jod, vorzugsweise Fluor oder Chlor;
- C₁-C₄-Alkyl für: Methyl, Ethyl, n-Propyl, 1-Methylethyl, n-Butyl, 1-Methylpropyl, 2-Methylpropyl oder 1,1-Dimethylethyl, vorzugsweise Methyl oder Ethyl;
- C₁-C₄-Halogenalkyl für: C₁-C₄-Alkyl wie vorstehend genannt, das partiell oder vollständig durch Fluor, Chlor und/oder Brom substituiert ist, also z.B. für Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 3-Chlorpropyl, vorzugsweise Trifluormethyl, Difluormethyl, 2,2,2-Trifluorethyl oder Pentafluorethyl;
- C₁-C₄-Alkoxy für: Methoxy, Ethoxy, n-Propoxy, 1-Methylethoxy, n-Butoxy, 1-Methyl-propoxy, 2-Methylpropoxy oder 1,1-Dimethylethoxy, vorzugsweise Methoxy oder Ethoxy;
- C₁-C₄-Halogenalkoxy für: C₁-C₄-Alkoxy wie vorstehend genannt, das partiell oder vollständig durch Fluor, Chlor und/oder Brom substituiert ist, also z.B, Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy oder 3-Chlorpropoxy, vorzugsweise Difluormethoxy oder Trifluormethoxy;
- C₁-C₄-Alkylcarbonyl für: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl oder 1,1-Dimethylethylcarbonyl, vorzugsweise Methylcarbonyl;
- C₁-C₄-Alkoxycarbonyl für: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl oder 1,1-Dimethylethoxycarbonyl, vorzugsweise Methoxycarbonyl, Ethoxycarbonyl oder 1,1-Dimethylethoxycarbonyl;
- C₁-C₄-Alkylamino für: Methylamino, Ethylamino, n-Propylamino, 1-Methylethylamino, n-Butylamino, 1-Methylpropylamino, 2-Methylpropylamino oder 1,1-Dimethylethylamino, vorzugsweise Methylamino oder Ethylamino;
- Di-(C₁-C₄-alkyl)amino für: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylprogyl)amino oder N-(1,1-Dimethylethyl)-N-(2-methylpropyl)amino, vorzugsweise Dimethylamino oder Diethylamino.

Heteroaryl steht vorzugsweise für einen 5- oder 6-gliedrigen aromatischen Heteroryclus mit einem Sauerstoff- und einem Schwefelatom oder ein 5- oder 6-gliedriger aromatischer Heterocyclus mit 1 bis 3 Heteroatomen, ausgewählt aus einer Gruppe bestehend aus 3 Stickstoffatomen und einem Sauerstoff- oder Schwefelatom: wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere 2-Pridinyl, 3-Pyridinyl und 4-Pyridinyl.

Die 5- oder 6-gliedrigen Heteroarylringe tragen mindestens einen Substituenten, höchstens jedoch so viele, wie substituierbare Atome vorhanden sind.

Im Hinblick auf die Verwendung der 2-Aroylcyclohexandione I als Herbizide oder zur Regulierung des Pflanzenwachstums bedeuten
- A: Methylen;
- R¹: vorzugsweise Phenyl, wobei das Phenyl mit höchstens vier, insbesondere einen oder zwei Substituenten substituiert sein kann, wobei jeder Substituent ausgewählt ist aus der Gruppe bestehend aus Halogen, Nitro, -N=N-Ph, -S(O)ₘR⁸, (C₁-C₄-Alkoxy)carbonyl, -N(R⁹)-COR¹⁰, -N(R⁹)-SO₂-R¹¹, -SO₂-N(R⁹)R¹⁰, C₁-C₄-Alkyl, C₁-C₄-Alkoxy, C₁-C₄-Halogenalkyl und C₁-C₄-Halogenalkoxy;
mit der Maßgabe, daß mindestens ein Substituent am Phenyl einen Rest -S(O)ₘR⁸ mit m=2 und R⁸ C₁-C₄-Alkyl bedeutet;
Ph steht vorzugsweise für Phenyl, das unsubstituiert sein oder gewünschtenfalls ein bis drei Substituenten tragen kann, ausgewählt aus der Gruppe bestehend aus Halogen, Cyano, Nitro, -S(O)ₙR²², C₁-C₄-Alkoxycarbonyl, -SO₂-N(R²³)R²⁴, -N(R²³)-COR²⁴, -N(R²³)-SO₂R²⁵, C₁-C₄-Alkyl, C₁-C₄-Alkoxy, C₁-C₄-Halogenalkyl oder C₁-C₄-Halogenalkoxy;
- m und n: stehen unabhängig voneinander für 0, 1 oder 2;
- R⁸ und R²²: stehen vorzugsweise für C₁-C₄-Alkyl oder C₁-C₄-Halogenalkyl;
- R⁹, R¹⁰, R²³ und R²⁴: stehen vorzugsweise für Wasserstoff, C₁-C₄-Alkyl, Phenyl oder Phenyl, das ein bis drei Reste trägt, ausgewählt aus der Gruppe Halogen, C₁-C₄-Alkyl, C₁-C₄-Halogenalkyl und C₁-C₄-Alkoxy;
- R¹¹ und R²⁵: stehen vorzugsweise für C₁-C₄-Alkyl oder C₁-C₄-Halogenalkyl;
- R² bis R⁶: vorzugsweise Wasserstoff und
- R⁷: vorzugsweise Methyl oder Ethyl.

Aufgrund ihres sauren Charakters können die erfindungsgemäßen 2-Aroylcyclohexandione I basische Salze oder Enolester bilden, wobei es auf die Art des Salzes oder Esters im allgemeinen nicht ankommt.

Als Salze der Verbindungen der Formel I kommen landwirtschaftlich brauchbare Salze, beispielsweise Alkalimetallsalze, insbesondere das Natrium- oder Kaliumsalz, Erdalkalimetallsalze insbesondere das Calcium-, Magnesium- oder Bariumsalz, Mangan-, Kupfer-, Zink- oder Eisensalz sowie Ammonium-, Phophonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Unter landwirtschaftlich brauchbaren Estern sind die Ester von
- C₁-C₁₀-Fettsäuren, insbesondere (C₁-C₆-Alkyl)carbonsäuren wie Methylcarbonsäure (Essigsäure), Ethylcarbonsäure (Propionsäure), n-Propylcarbonsäure (Buttersäure), 1-Methylethylcarbonsäure (Isobuttersäure), n-Butylcarbonsäure, 1-Methylpropylcarbonsäure, 2-Methylpropylcarbonsäure, 1,1-Dimethylethylcarbonsäure, n-Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, n-Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsäure,
- C₁-C₁₀-Sulfonsäuren insbesondere C₁-C₆-Alkylsulfonsäuren wie Methylsulfonsäure, Ethylsulfonsäure, n-Propylsulfonsäure, 1-Methylethylsulfonsäure, n-Butylsulfonsäure, 1-Methylpropylsulfonsäure, 2-Methylpropylsulfonsäure, 1,1-Dimethylethylsulfonsäure, n-Pentylsulfonsäure, 1-Methylbutylsulfonsäure, 2-Methylbutylsulfonsäure, 3-Methylbutylsulfonsäure, 1,1-Dimethylpropylsulfonsäure, 1,2-Dimethylpropylsulfonsäure, 2,2-Dimethylpropylsulfonsäure, 1-Ethylpropylsulfonsäure, Benzolsulfonsäure sowie durch Halogen substituierte Benzolsulfonsäuren, n-Hexylsulfonsäure, 1-Methylpentylsulfonsäure, 2-Methylpentylsulfonsäure, 3-Methylpentylsulfonsäure, 4-Methylpentylsulfonsäure, 1,1-Dimethylbutylsulfonsäure, 1,2-Dimethylbutylsulfonsäure, 1,3-Dimethylbutylsulfonsäure, 2,2-Dimethylbutylsulfonsäure, 2,3-Dimethylbutylsulfonsäure, 3,3-Dimethylbutylsulfonsäure, 1-Ethylbutylsulfonsäure, 2-Ethylbutylsulfonsäure, 1,1,2-Trimethylpropylsulfonsäure, 1,2,2-Trimethylpropylsulfonsäure, 1-Ethyl-1-methylpropylsulfonsäure und 1-Ethyl-2-methylpropylsulfonsäure, und
- C₁-C₁₀-Phosphonsäuren, insbesondere C₁-C₆-Alkylphosphonsäuren wie Methylphosphonsäure, Ethylphosphonsäure, n-Propylphosphonsäure, 1-Methylethylphosphonsäure, n-Butylphosphonsäure, 1-Methylpropylphosphonsäure, 2-Methylpropylphosphonsäure, 1,1-Dimethylethylphosphonsäure, n-Pentylphosphonsäure, 1-Methylbutylphosphonsäure, 2-Methylbutylphosphonsäure, 3-Methylbutylphosphonsäure, 1,1-Dimethylpropylphosphonsäure, 1,2-Dimethylpropylphosphonsäure, 2,2-Dimethylpropylphosphonsäure, 1-Ethylpropylphosphonsäure, Benzolphosphonsäure sowie durch Halogen substituierte Benzolphosphonsäuren, n-Hexylphosphonsäure, 1-Methylpentylphosphonsäure, 2-Methylpentylphosphonsäure, 3-Methylpentylphosphonsäure, 4-Methylpentylphosphonsäure, 1,1-Dimethylbutylphosphonsäure, 1,2-Dimethylbutylphosphonsäure, 1,3-Dimethylbutylphosphonsäure, 2,2-Dimethylbutylphosphonsäure, 2,3-Dimethylbutylphosphonsäure, 3,3-Dimethylbutylphosphonsäure, 1-Ethylbutylphosphonsäure, 2-Ethylbutylphosphonsäure, 1,1,2-Trimethylpropylphosphonsäure, 1,2,2-Trimethylpropylphosphonsäure, 1-Ethyl-1-methylpropylphosphonsäure und 1-Ethyl-2-methylpropylphosphonsäure
zu verstehen.

Die 2-Aroylcyclohexandione I können in mehreren tautomeren Formen geschrieben werden, die alle von der Erfindung umfaßt werden:

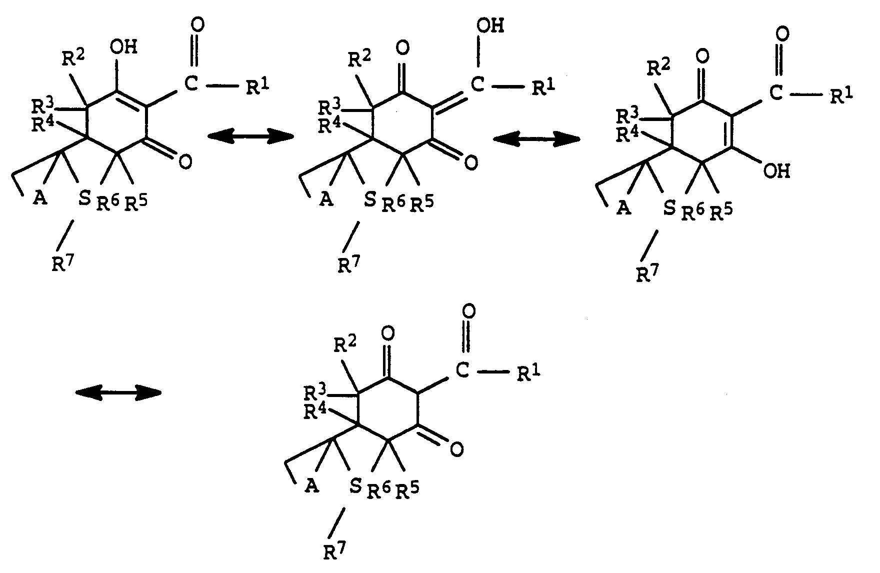

In der folgenden Tabelle 1 sind ganz besonders bevorzugte 2-Aroylcyclohexandione I aufgeführt (R² bis R⁶ bedeuten jeweils Wasserstoff):

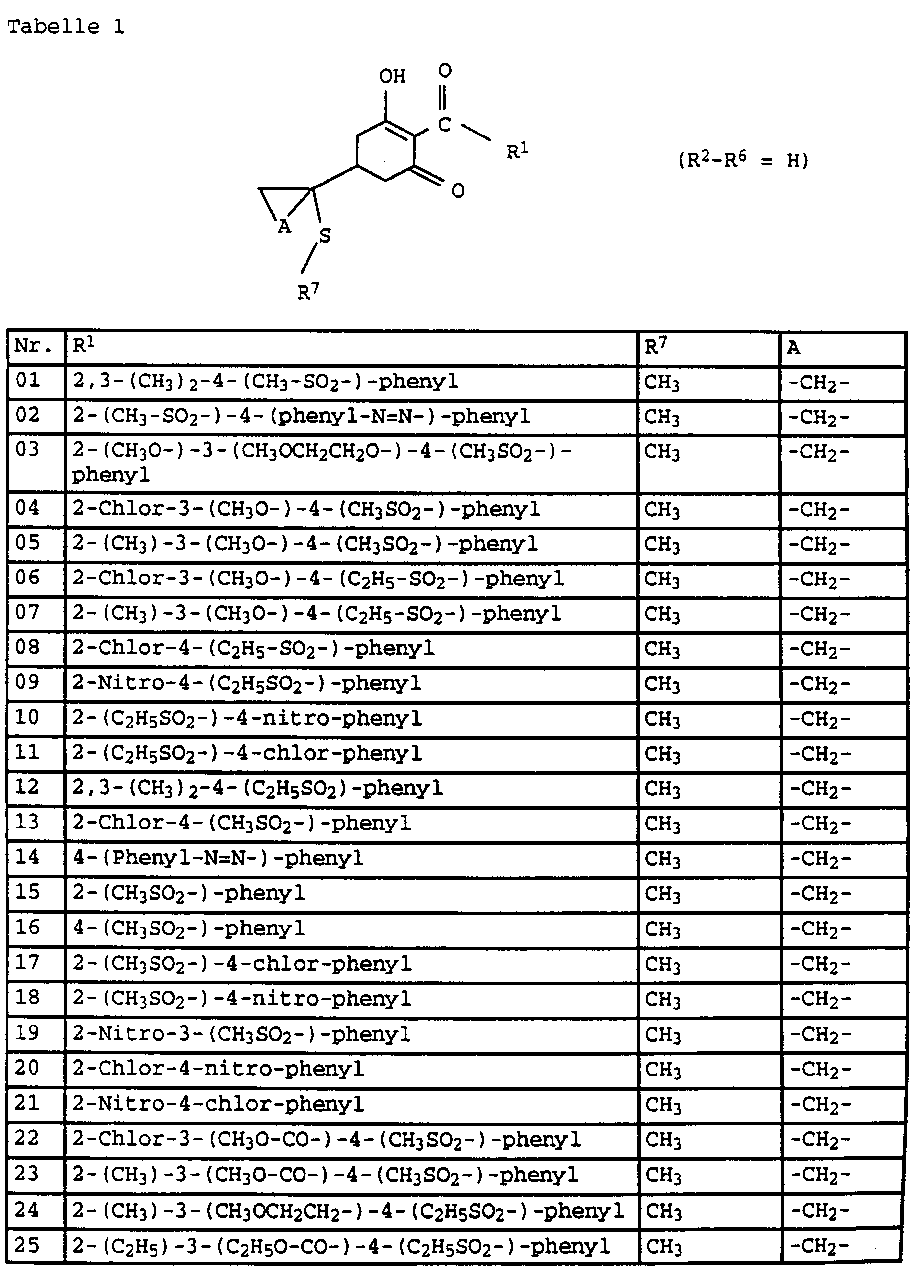

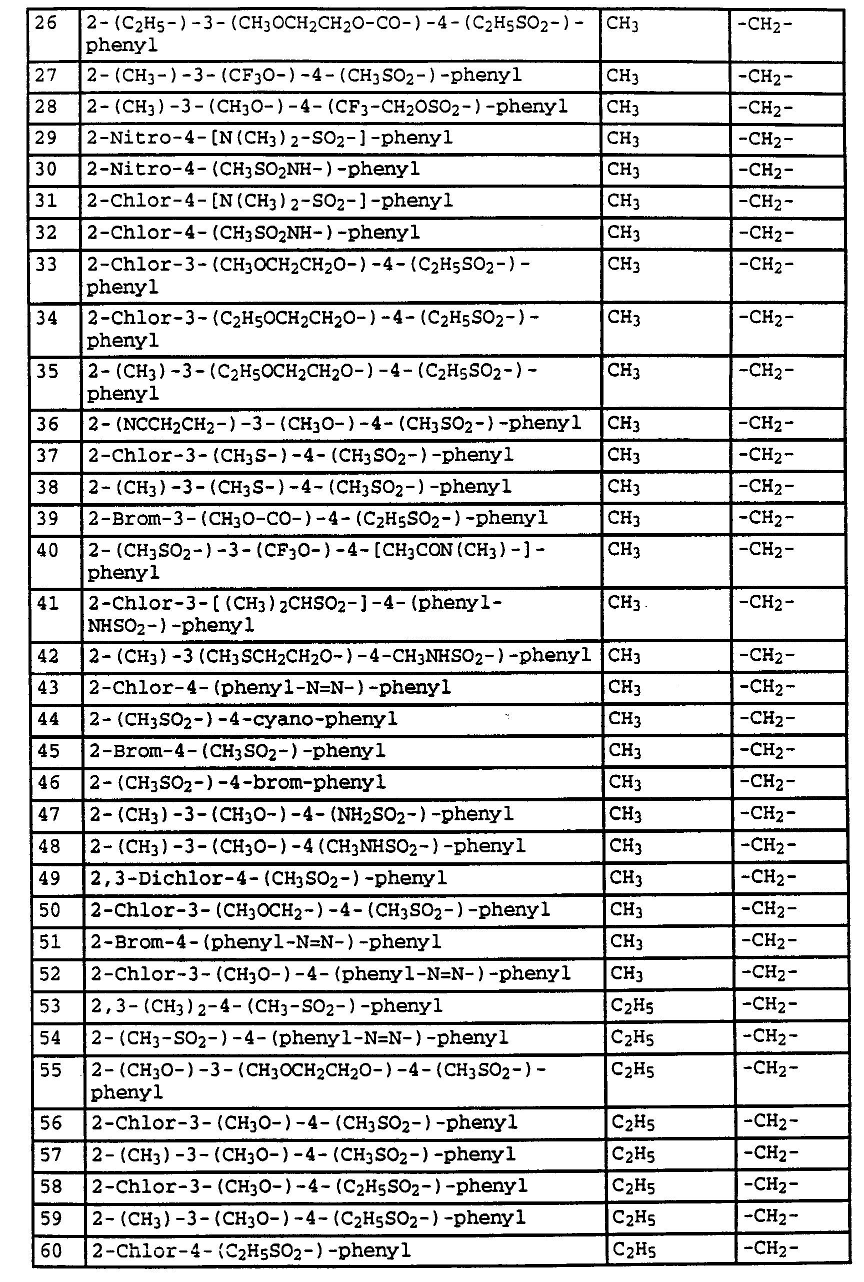

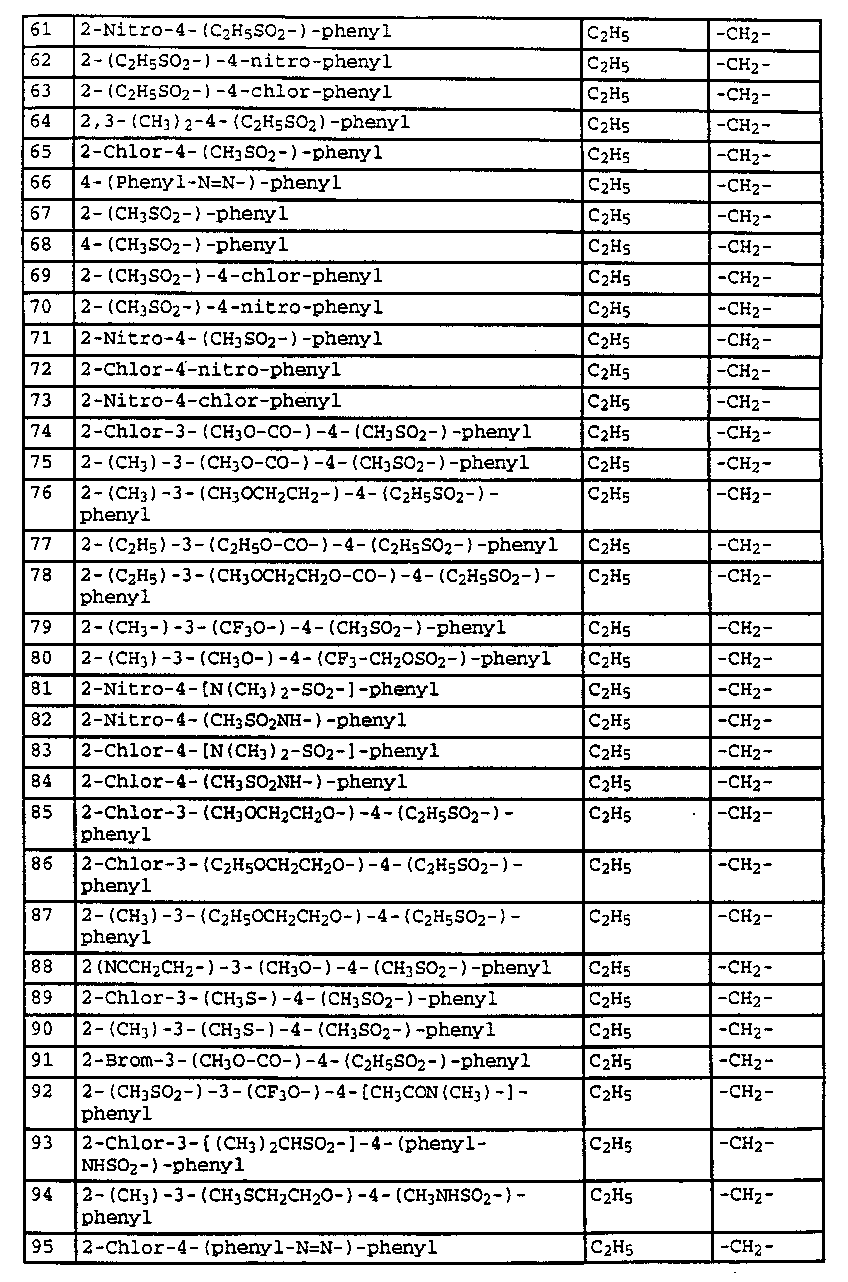

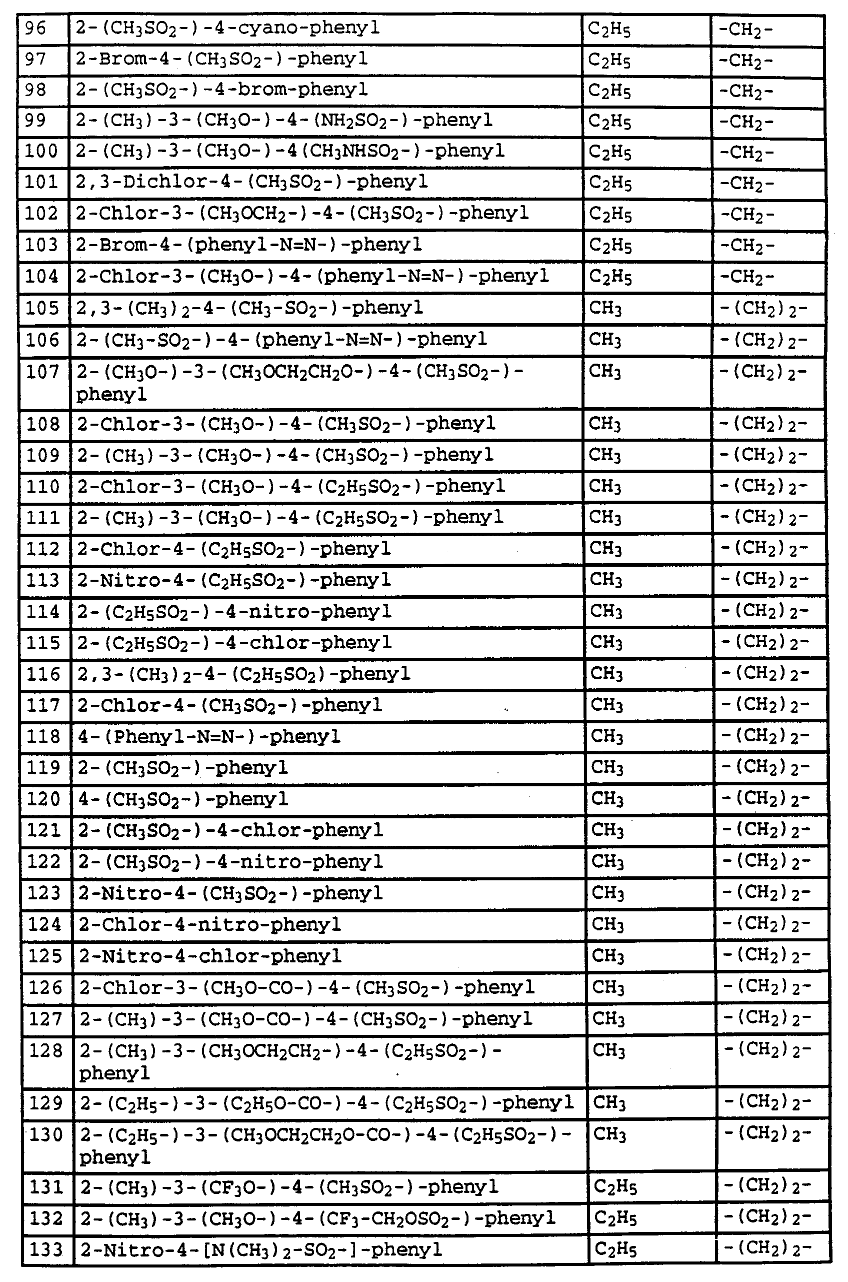

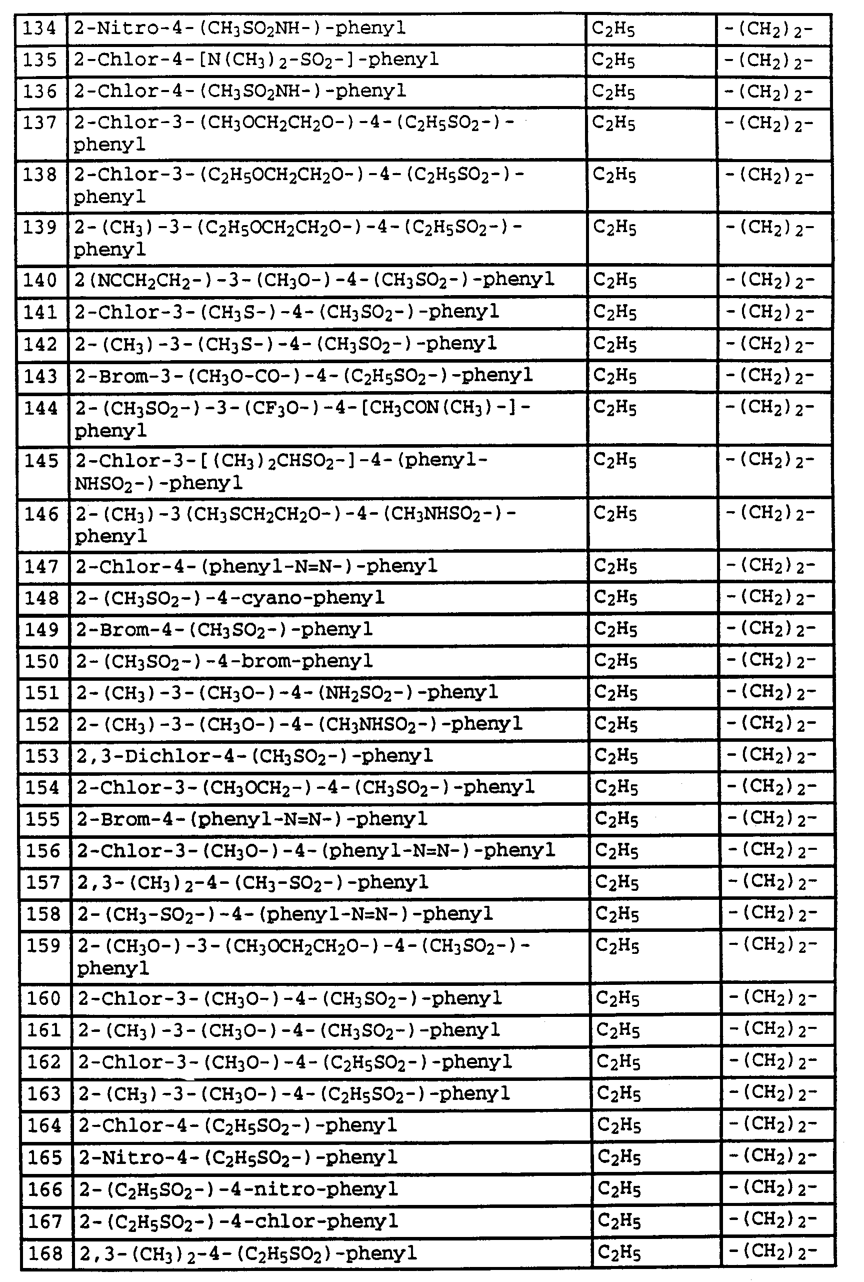

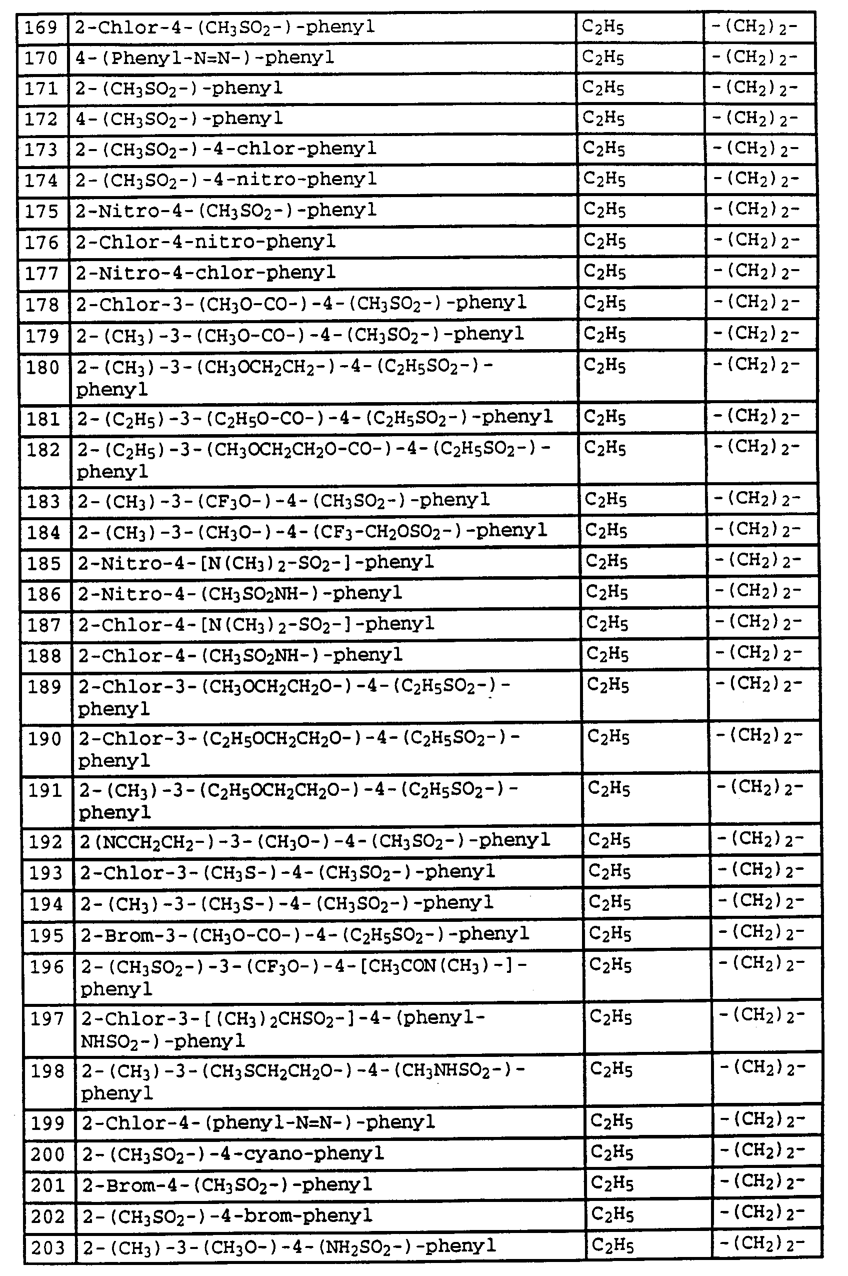

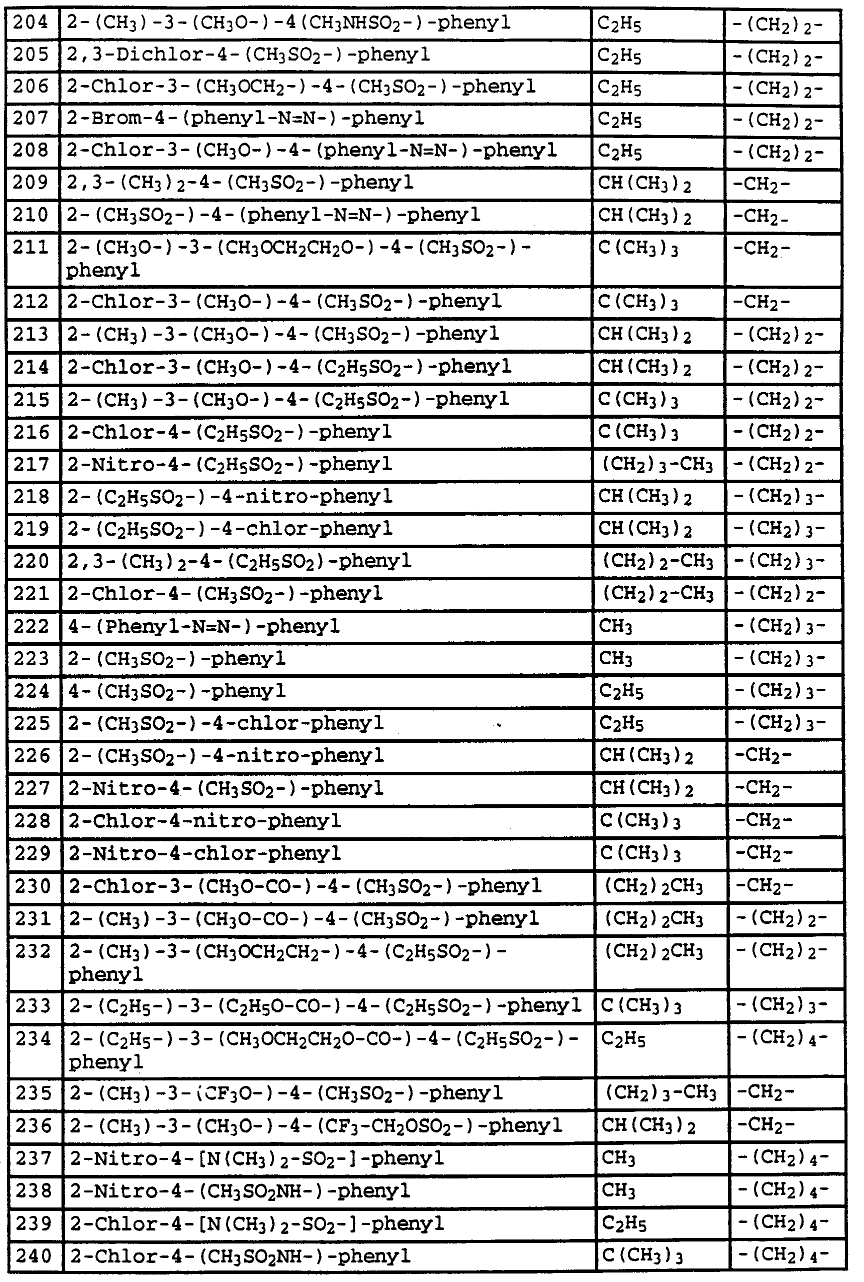

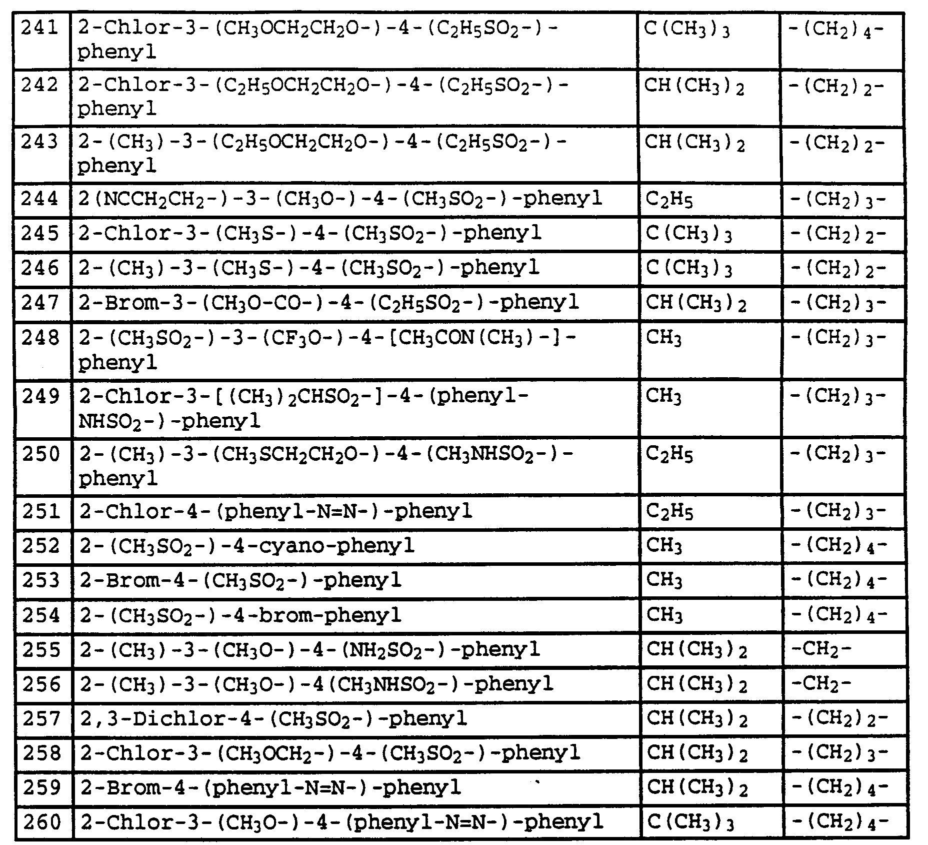

Die neuen 2-Aroylcyclohexandione I sind auf verschiedene Weise erhältlich, beispielsweise durch Umsetzung eines Cyclohexandions der Formel II mit einem Säurederivat der Formel III in Gegenwart einer Base (vgl. z.B. EP-A 186 118 und U.S. 4,695,673): 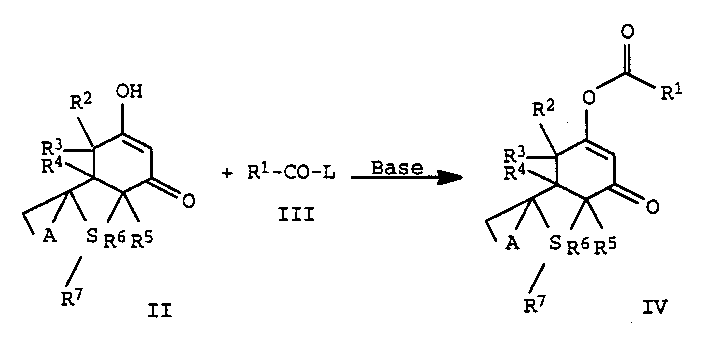 L steht für eine nukleophile Abgangsgruppe wie Chlorid, Bromid und Cyanid.

Die Reaktion wird in einem inerten Lösungsmittel zwischen 0°C und dem Siedepunkt des jeweiligen Lösungsmittels, vorzugsweise zwischen 0 und 20°C, durchgeführt.

Geeignete Lösungsmittel sind z.B. Chlorkohlenwasserstoffe wie Methylenchlorid, Chloroform und Dichlorethan, Ether wie Tetrahydrofuran, Dioxan und Methyltertiärbutylether, aromatische Kohlenwasserstoffe wie Benzol und Toluol, Ester wie Essigsäureethylester oder Amide wie Dimethylformamid.

Als Basen kommen beispielsweise tert. Amine wie Triethylamin, N-Methylmorpholin und Pyridin in Betracht.

Die Base wird vorteilhaft in etwa äquimolarer Menge, bezogen auf das Diketon II oder das Säurederivat III, eingesetzt.

Die Umsetzung kann auch in einem heterogenen System durchgeführt werden, wobei dann das Diketon II und die Base in wäßriger Lösung vorliegen, zu der das Säurederivat in einem mit Wasser nicht mischbaren Lösungsmittel, in Gegenwart eines Phasentransferkatalysators, gegeben wird.

Brauchbare Basen hierfür sind z.B. die Alkalimetall- und Erdalkalimetallhydroxide oder -carbonate wie Natriumhydroxid, Kaliumhydroxid, Natriumcarbonat, Kaliumcarbonat, Calciumcarbonat und Calciumhydroxid.

Beispiele für verwendbare Phasentransferkatalysatoren sind Ammonium-, Sulfonium- oder Phosphoniumsalze, wobei die Art des Anions von untergeordneter Bedeutung ist. Als zweckmäßig hat sich z.B. Benzyltrimethylammoniumhydroxid erwiesen.

Die erhaltenen Enolester der Formel IV werden dann in Gegenwart einer Cyanid-Quelle und einer Base in die gewünschten Verbindungen der Formel I umgelagert:

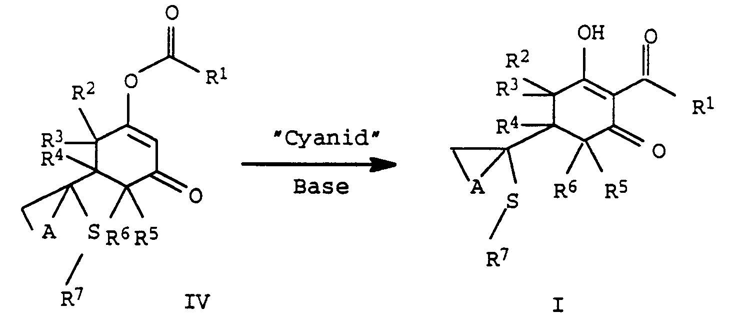

Die Umlagerung wird üblicherweise in einem inerten Lösungsmittel bei einer Temperatur zwischen 0°C und dem Siedepunkt des Lösungsmittels, vorzugsweise zwischen 20 und 40°C vorgenommen.

Geeignete Lösungsmittel sind z.B. Chlorkohlenwasserstoffe wie Methylenchlorid, Chloroform und Dichlorethan, Ether wie Tetrahydrofuran, Dioxan und Methyltertiärbutylether, aromatische Kohlenwasserstoffe wie Benzol und Toluol, Nitrile wie Acetonitril, Ester wie Essigsäureethylester, Ketone wie Aceton und Methylethylketon oder Amide wie Dimethylformamid.

Als Cyanidquellen haben sich z.B. Alkalimetallcyanide wie Natriumcyanid und Kaliumcyanid, Cyanhydrine wie Acetoncyanhydrin und Trialkylsilylcyanide wie Trimethylsilylcyanid als geeignet erwiesen.

Normalerweise ist eine katalytische Menge an Cyanid, etwa zwischen 1 und 10 Mol%, bezogen auf den Enolester IV, ausreichend. Brauchbare Basen sind z.B. Trialkylamine wie Triethylamin, Trialkanolamine wie Triethanolamin und Pyridin oder anorganische Basen wie Alkalimetallcarbonate und -phosphate.

Es hat sich als vorteilhaft erwiesen, die Base in einem Überschuß von 100 bis 400 Mol%, bezogen auf den Enolester IV, zuzusetzen.

Aus den nach diesem Verfahren erhaltenen Reaktionsgemischen können die 2-Aroylcyclohexandione I mittels üblicher Aufarbeitungsmethoden isoliert weren, beispielsweise durch Extraktion oder durch Kristallisation.

Besondere Bedingungen bezüglich des Drucks sind bei der Herstellung der Verbindungen I nicht zu beachten; im allgemeinen arbeitet man daher bei Normaldruck oder unter dem Eigendruck des jeweiligen Verdünnungsmittels.

Die Diketone der Formel II sind aus der EP-A 243 313 bekannt oder können auf die dort beschriebene Weise hergestellt werden. Die benötigten Säurederivate III sind bekannt oder können nach an sich bekannten Methoden erhalten werden {vgl. z.B. The Chemistry of Carboxylic Acids and Esters, S. Patai, editor, J. Wiley and Sons, New York, N.Y.(1969); Survey of Organic Synthesis, C.A. Buehler and D.F. Pearson, J. Wiley and Sons, (1970); Reagents for Organic Synthesis, Vol. I, L.F. Fieser and M. Fieser, S. 767-769 (1967)}.

Alkalimetallsalze der Verbindungen I können durch Behandeln von I mit Natrium- oder Kaliumhydroxid oder -alkoholat in wäßriger Lösung oder in einem organischen Lösungsmittel wie Methanol, Ethanol, Aceton, Toluol erhalten werden.

Andere Metallsalze, z. B. die Mangan-, Kupfer-, Zink-, Eisen-, Calcium-, Magnesium- und Bariumsalze, können aus den Natriumsalzen in üblicher Weise hergestellt werden, ebenso Ammonium-, Phosphonium-, Sulfonium- oder Sulfoxoniumsalze mittels Ammoniak, Phosphonium-, Sulfonium- oder Sulfoxoniumhydroxiden.

Die Ester der Verbindungen I sind ebenfalls in üblicher Weise erhältlich (vgl. z.B. EP-A 102 823 und EP-A 136 702).

Die 2-Aroylcyclohexandione I, deren Salze und Ester - bzw. die diese Verbindungen enthaltenden Mittel - können in Kulturen wie Weizen, Reis, Mais, Soja und Baumwolle Unkräuter und Schadgräser sehr gut bekämpfen, ohne die Kulturpflanzen zu schädigen. Dieser Effekt tritt vor allem bei niedrigen Aufwandmengen auf.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Ahwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew. %, vorzugsweise zwischen 0,5 und 90 Gew. %, Wirkstoff. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können weiterhin beispielsweise wie folgt formuliert werden:
I. 20 Gewichtsteile der Verbindung Nr. 1.4 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 1.5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 1.6 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 1.7 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser ser erthält man eine Spritzbrühe, die 0,1 Gew.% des Wirkstoffs enthält.
V. 3 Gewichtsteile des Wirkstoffs Nr. 1.19 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 1.32 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-FormaldehydKondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus lassen sich die Verbindungen I in Kulturen, die durch Züchtung und/oder mittels gentechnischer Methoden gegen die Wirkung von I weitgehend resistent gemacht wurden, einsetzen.

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig
a) von der Pflanzenart und -sorte,
b) von dem Zeitpunkt der Applikation, bezogen auf das Entwicklungsstadium der Pflanze, und von der Jahreszeit,
c) von dem Applikationsort und -verfahren (z. B. Samenbeize, Bodenbehandlung, Blattapplikation oder Stamminjektion bei Bäumen),
d) von klimatischen Faktoren (z. B. Temperatur, Niederschlagsmenge, außerdem auch Tageslänge und Lichtintensität),
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchte zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stomata reduziert wird,
   - eine dickere Epidermis und Cuticula ausgebildet werden,
   - die Durchwurzelung des Bodens verbessert wird und
   - das Mikroklima im Pflanzenbestand durch einen kompakteren Wuchs günstig beeinflußt wird.

Besonders gut eignen sich die Verbindungen I zur Halmverkürzung von Kulturpflanzen wie Gerste, Raps und Weizen.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Zur Verbreiterung des Wirkungsspektrums und zur Erzielung synergistischer Effekte können die 2-Aroylcyclohexandione I mit zahlreichen Vertretern anderer herbizider oder wachstums regulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht. Bei Wachstumsregulatoren kommen insbesondere Chlormequatchlorid, Ethephon und Mepiquatchlorid in Frage.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösung, welche zur Behebung von Ernährungs- und Spurenelementmängeln eingesetzt werden. Es können auch nichtphytotoxische Öle und Ölkonzentrate zugesetzt werden.

### Herstellungsbeispiel

### 2-(2-Chlor-4-methylsulfonylbenzoyl)-5-(1-methylthiocyclopropyl)-cyclohexan-1,3-dion

Zu einer Lösung von 1,6 g (3,9 mmol) 3-(2-Chlor-4-methylsulfonylbenzoyloxy)-5-(1-methylthiocyclopropyl)-cyclohex-2-enon in 40 ml wasserfreiem Acetonitril wurden 0,2 g (2,4 mmol) Acetoncyanhydrin gegeben, wonach man bei ca. 20 °C eine Lösung von 2,0 g (20 mmol) Triethylamin in 10 ml Acetonitril zutropfte. Das Reaktionsgemisch wurde anschließend 3 Stunden bei etwa 20 °C gerührt und dann auf 30 ml 3 gew.-%ige kalte Salzsäure gegossen. Nach Extraktion mit 80 ml Methyl-tert.-butylether wurde die organische Phase viermal mit je 50 ml 5 gew.%-iger wässriger Kaliumcarbonat-Lösung extrahiert. Die vereinigten alkalischen Extrakte wurden unter Eiskühlung mit konzentrierter Salzsäure bis zu einem pH-wert von 1 angesäuert. Aus dieser Lösung erhielt man das Wertprodukt durch zweimalige Extraktion mit 100 ml Methyl-tert.-butylether, der schließlich getrocknet und eingeengt wurde. Ausbeute: 1,2 g (75 %); Smp.: 148-152 °C.

### Vorstufe:

Zu einer Lösung von 1 g (5 mmol) 5-(1-Methylthiocyclopropyl)-cyclohexan-1,3-dion in 20 ml wasserfreiem Tetrahydrofuran wurden unter Ausschluß von Feuchtigkeit 0,5 g (5 mmol) Triethylamin gegeben, wonach man bei 0 °C eine Lösung von 1,3 g (5 mmol) 2-Chlor-4-methylsulfonylbenzoesäurechlorid in 10 ml wasserfreiem Tetrahydrofuran zutropfte. Nach beendeter Zugabe wurde die Reaktionsmischung noch 12 Stunden bei ca. 20 °C gerührt und dann mit 80 ml Methylenchlorid versetzt. Zur Aufarbeitung wurde die organische Phase mit 40 ml Wasser, 40 ml halbkonzentrierter wässriger Natriumcarbonat-Lösung und nochmals mit Wasser gewaschen, schließlich mit Natirumsulfat getrocknet und eingeengt. Ausbeute: 1,7 g (81 %) 3-(2-Chlor-4-methylsulfonylbenzoyloxy)-5-(1-methylthiocyclopropyl)-cyclohex-2-enon.

In der folgenden Tabelle 2 sind noch weitere 2-Aroylcyclohexandione I aufgeführt, die auf die gleiche Weise hergestellt wurden oder herstellbar sind:

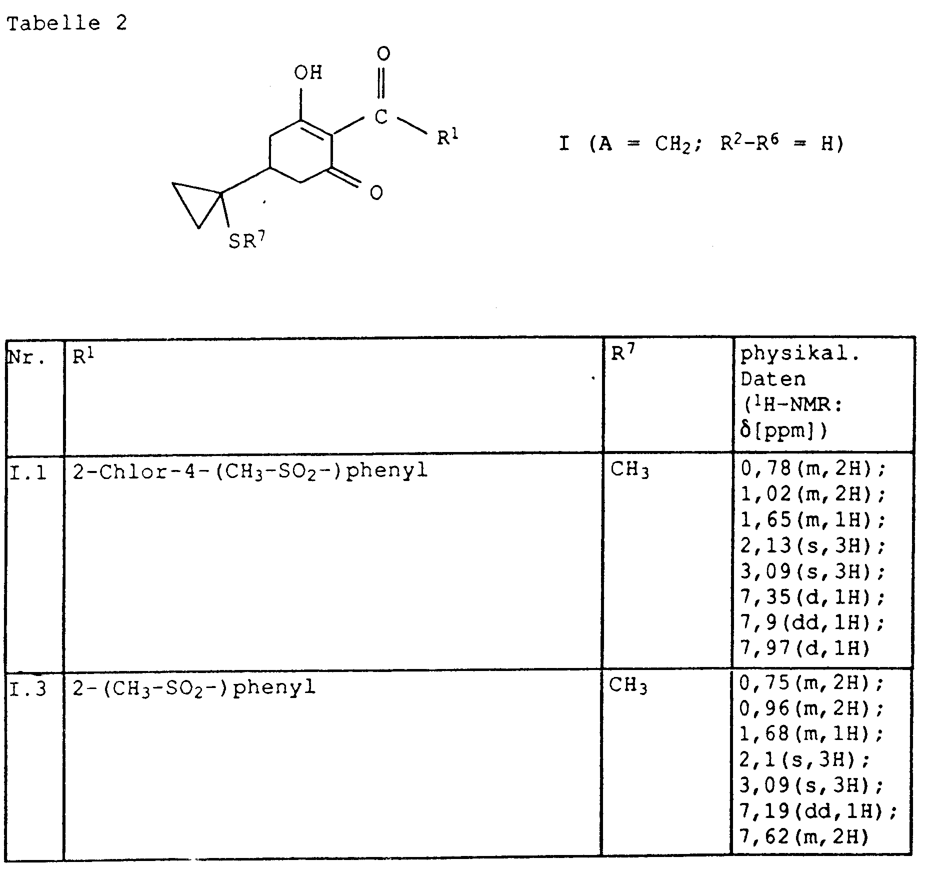

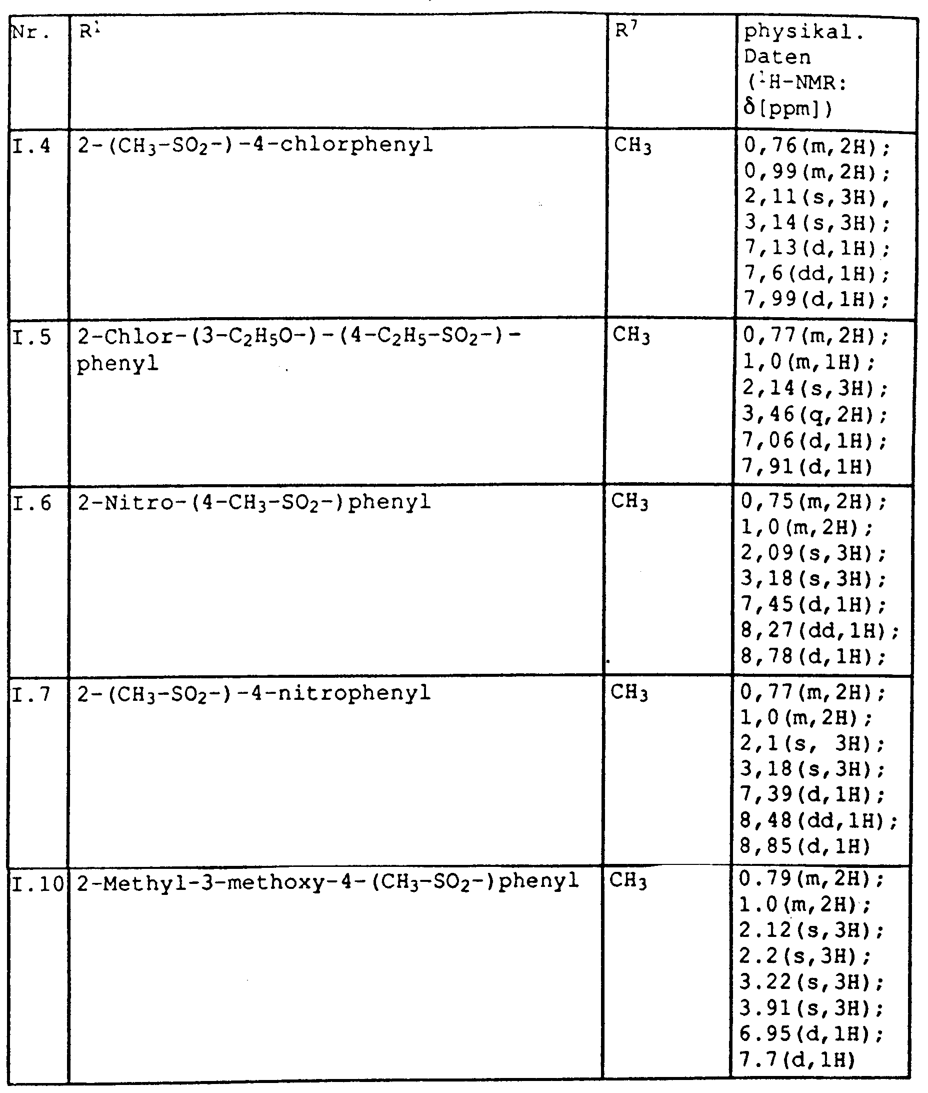

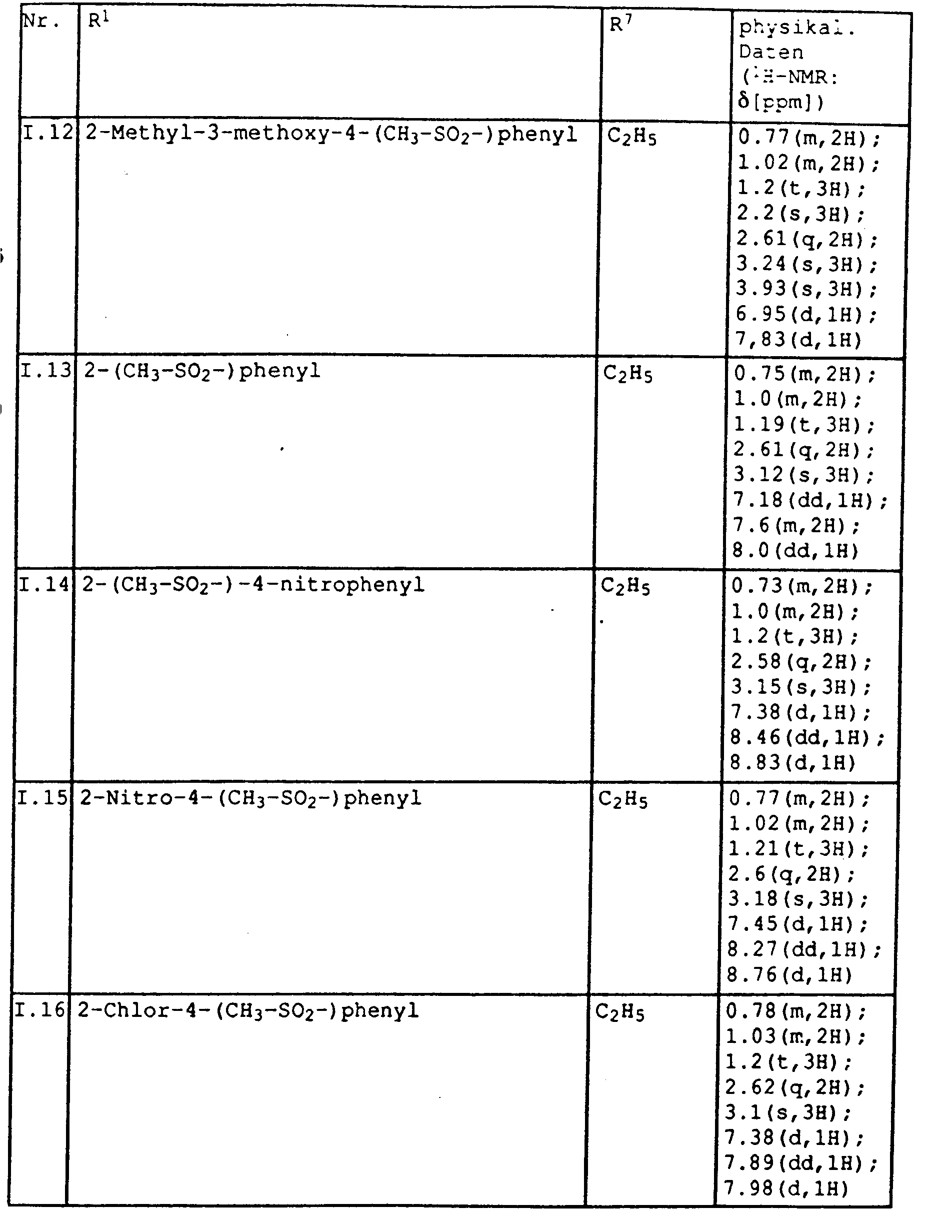

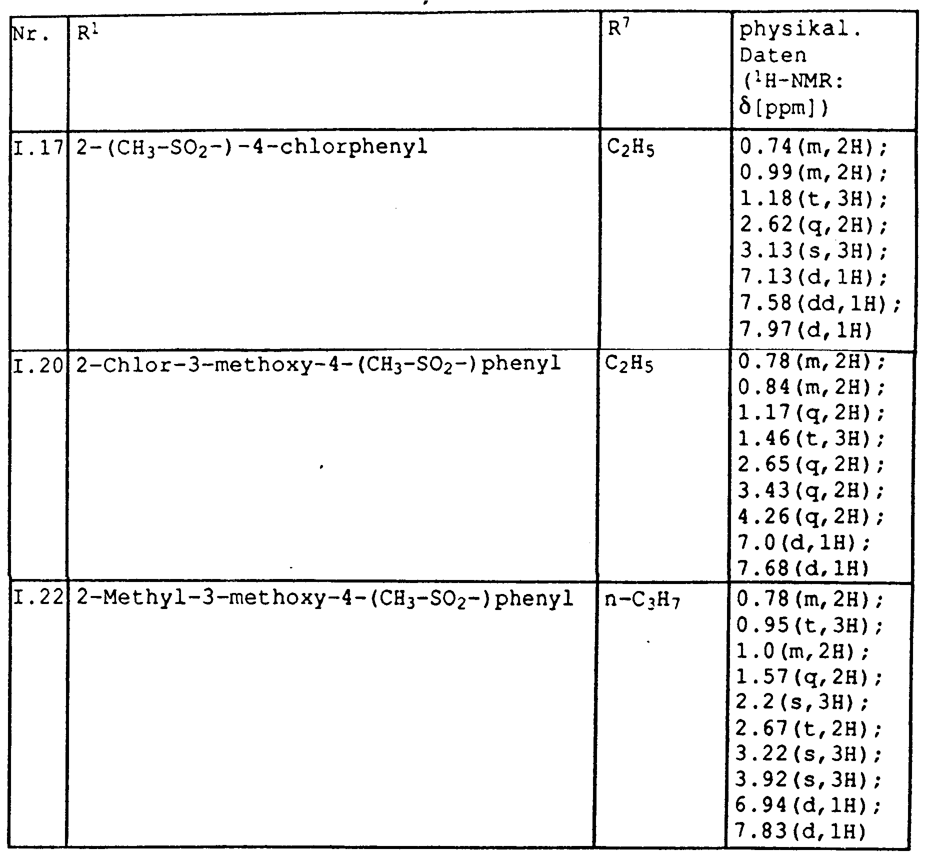

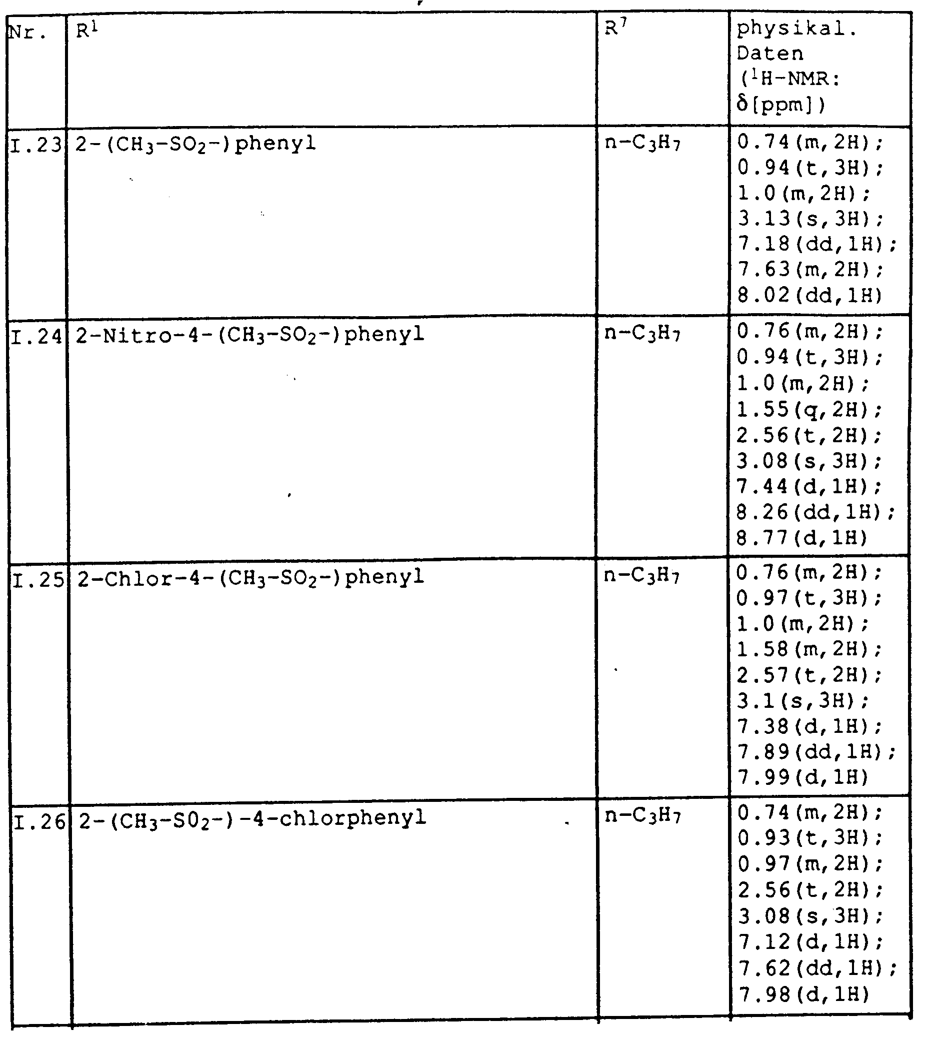

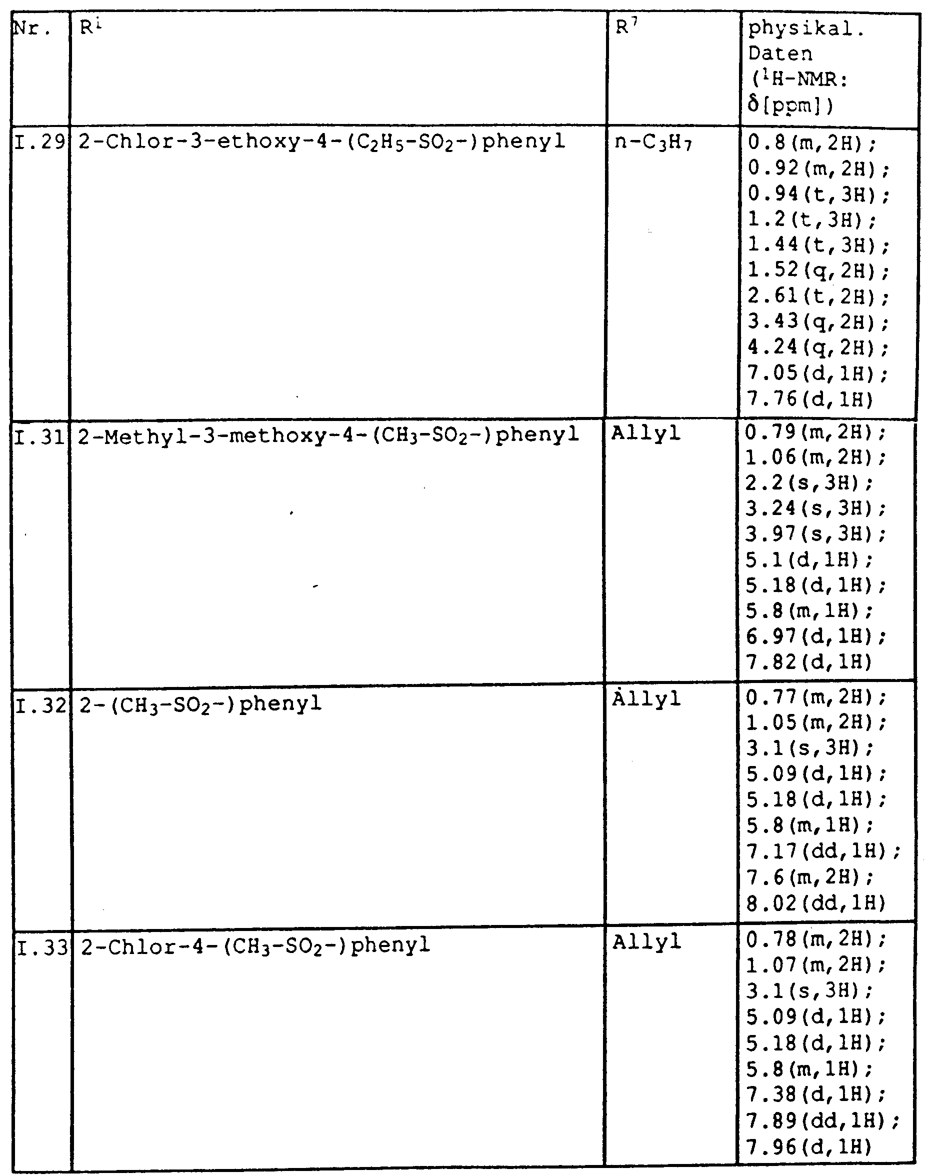

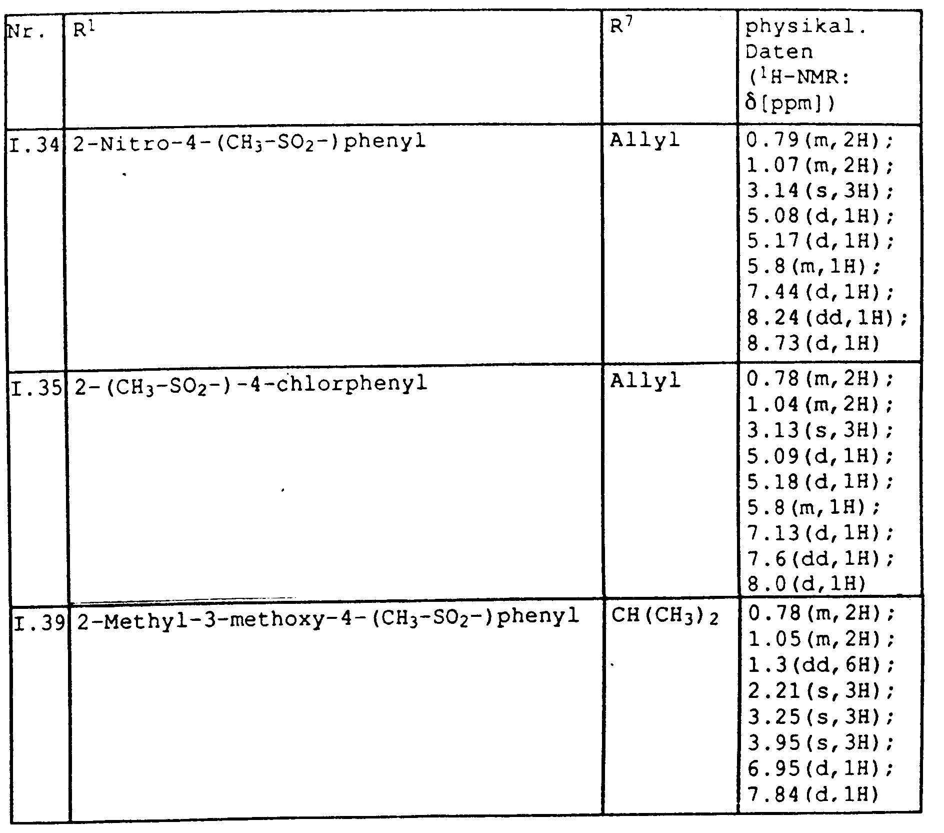

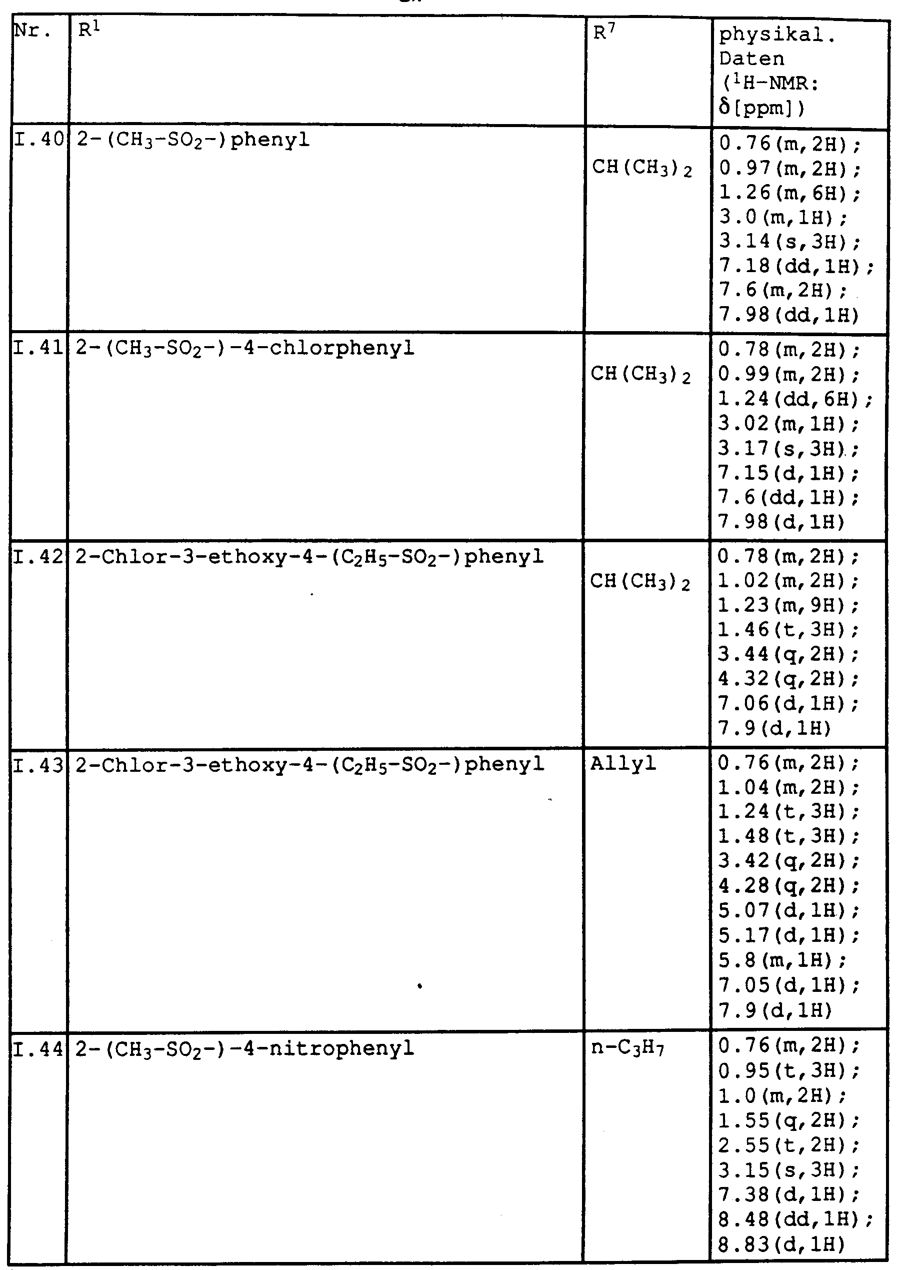

### Anwendungsbeispieie

Die herbizide und wachstumsregulatorische Wirkung der 2-Aroylcyclohexandione der Formel I ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 3,0 kg/ha a.S.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die in den Gewächshausversuchen verwendeten Pflanzen setzten sich aus folgenden Arten zusammen:

| Lateinischer Name | Deutscher Name | Englischer Name |
|---|---|---|
| Bromus inermis | unbegrannte Trespe | smooth broome |
| Centaurea cyanus | Kornblume | cornflower |
| Echinochloa crus-galli | Hühnerhirse | barnyardgrass |

Das Ergebnis zeigte, daß mit den Verbindungen Nr. 1.4 und 1.5 unerwünschte Pflanzen sehr gut bekämpft werden können.

## Patentansprüche

1. 2-Aroylcyclohexandione der Formel I 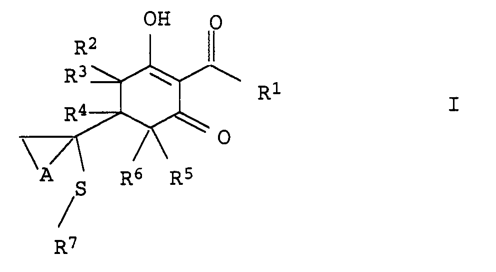 in der die Variablen folgende Bedeutung haben:
A Methylen
R¹ Phenyl, wobei Phenyl mit höchstens vier Substituenten substituiert sein kann, jeweils ausgewählt aus der Gruppe bestehend aus Halogen, Cyano, Nitro, -N=N-Ph, -S(O)ₘR⁸, (C₁-C₄-Alkoxy)carbonyl, -SO₂-N(R⁹)R¹⁰, -N(R⁹)-COR¹⁰, -N(R⁹)-SO₂R¹¹, C₁-C₄-Alkyl, C₁-C₄-Alkoxy, C₁-C₄-Halogenalkyl, C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano,
Ph steht für Phenyl, das unsubstituiert sein oder gewünschtenfalls ein bis drei Substituenten tragen kann, ausgewählt aus der Gruppe bestehend aus Halogen, Cyano, Nitro, -S(O)ₙR²², (C₁-C₄-Alkoxy)carbonyl, -SO₂-N(R²³)R²⁴, -N(R²³)-COR²⁴, -N(R²³)-SO₂R²⁵, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano;
m,n stehen jeweils für 0, 1 oder 2;
R⁸, R²² stehen unabhängig voneinander für C₁-C₄-Alkyl oder C₁-C₄-Halogenalkyl, wobei diese Gruppen ein oder zwei C₁-C₄-Alkoxy, C₁-C₄-Alkylthio- und/oder Cyanoreste tragen können;
R⁹, R¹⁰, R²³, R²⁴ stehen unabhängig voneinander für Wasserstoff, C₁-C₄-Alkyl, C₁-C₄-Halogenalkyl, Phenyl oder Phenyl, das ein bis drei Reste trägt, ausgewählt aus der Gruppe Halgen, C₁-C₄-Alkyl und C₁-C₄-Alkoxy;
R¹¹, R²⁵ stehen unabhängig voneinander für C₁-C₄-Alkyl oder C₁-C₄-Halogenalkyl, wobei diese Gruppen ein oder zwei Cyano-, Phenyl-und/oder Benzylreste tragen können;
R², R³, R⁴, R⁵ unabhängig voneinander Wasserstoff oder C₁-C₄-Alkyl;
R⁶ Wasserstoff, C₁-C₄-Alkyl oder (C₁-C₄-Alkoxy)carbonyl;
R⁷ C₁-C₄-Alkyl;
mit der Maßgabe, daß mindestens ein Substituent am Phenyl einen Rest -S(O)ₘR⁸ mit m=2 und R⁸ C₁-C₄-Alkyl bedeutet, sowie deren landwirtschaftlich brauchbaren Salze, die Ester von I mit C₁-C₁₀-Carbonsäuren oder anorganischen Säuren.

2. Verfahren zur Herstellung von 2-Aroylcyclohexandionen der Formel I gemäß Anspruch 1, dadurch gekennzeichnet, daß man eine Verbindung der Formel II 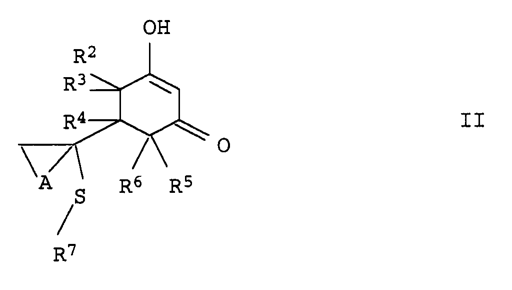 mit einem Säurechlorid der Formel III
R¹-CO-L III
in der L für eine Abgangsgruppe steht,
in Gegenwart einer Base in einem inerten Lösungsmittel umsetzt,
und den hierbei erhaltenen Enolester der Formel IV 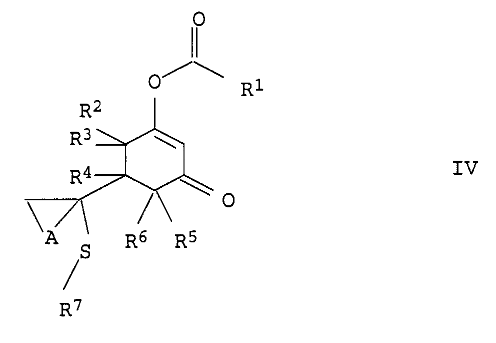 in Gegenwart einer Cyanid-Quelle und einer Base in einem inerten Lösungsmittel umgelagert.

3. Verwendung der 2-Aroylcyclohexandione I, deren landwirtschaftlich brauchbaren Salze und Ester, gemaß Anspruch 1, als Herbizide oder zur Regulierung des Pflanzenwachstums.

4. Herbizides Mittel, enthaltend eine herbizid wirksame Menge mindestens eines 2-Aroylcyclohexandions der Formel I oder ein landwirtschaftlich brauchbares Salz oder einen Ester von I, gemäß Anspruch 1, und mindestens einen inerten flüssigen und/oder festen Trägerstoff sowie gewünschtenfalls mindestens ein Adjuvents.

5. Verfahren zur Herstellung von herbizid wirksamen Mitteln, dadurch gekennzeichnet, daß man eine herbizid wirksame Menge mindestens eines 2-Aroylcyclohexandions der Formel I oder eines Salzes oder Esters von I, gemäß Anspruch 1, und mindestens einen inerten flüssigen und/oder festen Trägerstoff sowie gewünschtenfalls mindestens ein Adjuvants mischt.

6. Verfahren zur Bekämpfung von unerwünschtem Pflanzenwuchs, dadurch gekennzeichnet, daß man eine herbizid wirksame Menge mindestens eines 2-Aroylcyclohexandions der Formel I oder ein landwirtschaftlich brauchbares Salz oder einen Ester von I, gemäß Anspruch 1, auf Pflanzen, deren Lebensraum oder auf Saatgut einwirken läßt.

7. Mittel zur Regulierung des Pflanzenwachstums, enthaltend inerte Trägerstoffe und eine zum Regulieren des Pflanzenwachstums ausreichende Menge mindestens eines 2-Aroylcyclohexandions der Formel I oder eines Salzes oder Esters von I, gemäß Anspruch 1.

8. Verfahren zur Herstellung von Mitteln zur Regulierung des Pflanzenwachstums, dadurch gekennzeichnet, daß man eine zum Regulieren des Pflanzenwachstums ausreichende Menge mindestens eines 2-Aroylcyclohexandions der Formel I oder eines Salzes oder Esters von I, gemäß Anspruch 1, und mindestens einen inerten flüssigen und/oder festen Trägerstoff sowie gewünschtenfalls mindestens ein Adjuvants mischt.

9. Verfahren zur Regulierung des Pflanzenwachstums, dadurch gekennzeichnet, daß man eine zum Regulieren des Pflanzenwachstums ausreichende Menge mindestens eines 2-Aroylcyclohexandions der Formel I oder eines landwirtschaftlich brauchbaren Salzes oder Esters von I, gemäß Anspruch 1, auf Pflanzen, deren Lebensraum oder auf das Saatgut der Pflanzen einwirken läßt.

## Claims

1. A 2-aroylcyclohexanedione of the formula I 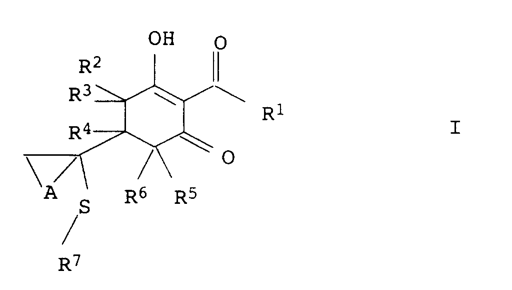 where
A is methylene;
R¹ is phenyl, it being possible for phenyl to be substituted by not more than four substituents, each selected from the group consisting of halogen, cyano, nitro, -N=N-Ph, -S(O)ₘR⁸, (C₁-C₄-alkoxy)carbonyl, -SO₂-N(R⁹)R¹⁰, -N(R⁹)-COR¹⁰, -N(R⁹)-SO₂R¹¹, C₁-C₄-alkyl, C₁-C₄-alkoxy, C₁-C₄-haloalkyl or C₁-C₄-haloalkoxy, where the four last-mentioned radicals in turn may carry one or two of the following substituents: C₁-C₄-alkoxy, C₁-C₄-alkylthio or cyano,
Ph is phenyl which may be unsubstituted or, if desired, may carry from one to three substituents selected from the group consisting of halogen, cyano, nitro, -S(O)ₙR²², (C₁-C₄-alkoxy)carbonyl, -SO₂-N(R²³)R²⁴, -N(R²³)-COR²⁴, -N(R²³)-SO₂R²⁵, C₁-C₄-alkyl, C₁-C₄-alkoxy, C₁-C₄-haloalkyl and C₁-C₄-haloalkoxy, where the four last-mentioned radicals in turn may carry one or two of the following substituents: C₁-C₄-alkoxy, C₁-C₄-alkylthio or cyano;
m and n are each 0, 1 or 2;
R⁸ and R²² independently of one another are each C₁-C₄-alkyl or C₁-C₄-haloalkyl, where these groups may carry one or two C₁-C₄-alkoxy, C₁-C₄-alkylthio or cyano radicals;
R⁹, R¹⁰, R²³ and R²⁴ independently of one another are each hydrogen, C₁-C₄-alkyl, C₁-C₄-haloalkyl, phenyl or phenyl which carries from one to three radicals selected from the group consisting of halogen, C₁-C₄-alkyl and C₁-C₄-alkoxy;
R¹¹ and R²⁵ independently of one another are each C₁-C₄-alkyl or C₁-C₄-haloalkyl, where these groups may carry one or two cyano, phenyl or benzyl radicals;
R², R³, R⁴ and R⁵ independently of one another are each hydrogen or C₁-C₄-alkyl;
R⁶ is hydrogen, C₁-C₄-alkyl or (C₁-C₄-alkoxy)carbonyl;
R⁷ is C₁-C₄-alkyl,
with the proviso that at least one substituent on the phenyl is a radical -S(O)ₘR⁸ where m is 2 and R⁸ is C₁-C₄-alkyl, and the agriculturally useful salts thereof and the esters of I with C₁-C₁₀-carboxylic acids or inorganic acids.

2. A process for the preparation of 2-aroylcyclohexanediones of the formula I as claimed in claim 1, which comprises reacting a compound of the formula II 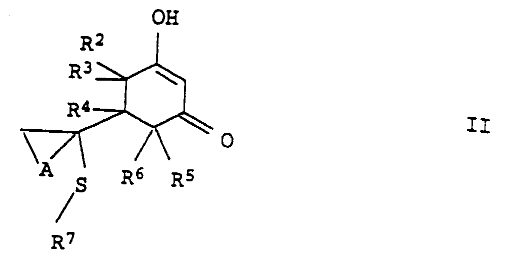 with an acid chloride of the formula III
R¹-CO-L (III)
where L is a leaving group,
in the presence of a base in an inert solvent, and rearranging the resulting enol ester of the formula IV 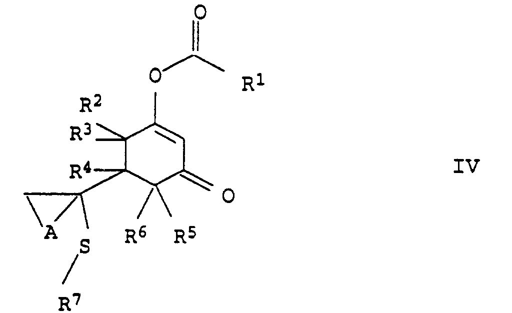 in the presence of a source of cyanide and of a base in an inert solvent.

3. The use of the 2-aroylcyclohexanediones I, and agriculturally useful salts and esters thereof, as claimed in claim 1, as herbicides or for the regulation of plant growth.

4. A herbicide containing a herbicidal amount of at least one 2-aroylcyclohexanedione of the formula I or an agriculturally useful salt or an ester of I, as claimed in claim 1, and at least one inert liquid or solid carrier and, if desired, at least one adjuvant.

5. A process for the preparation of a herbicide, wherein a herbicidal amount of at least one 2-aroylcyclohexanedione of the formula I or of a salt or ester of I, as claimed in claim 1, and at least one inert liquid or solid carrier and, if desired, at least one adjuvant are mixed.

6. A method for controlling undesirable plant growth, wherein a herbicidal amount of at least one 2-aroylcyclohexanedione of the formula I or an agriculturally useful salt or an ester of I, as claimed in claim 1, is allowed to act on plants or their habitat or on seed.

7. A plant growth regulator containing inert carriers and an amount, sufficient for regulating plant growth, of at least one 2-aroylcyclohexanedione of the formula I or of a salt or ester of I, as claimed in claim 1.

8. A process for the preparation of a plant growth regulator, wherein an amount, sufficient for regulating plant growth, of at least one 2-aroylcyclohexanedione of the formula I or of a salt or ester of I, as claimed in claim 1, and at least one inert liquid or solid carrier and, if desired, at least one adjuvant are mixed.

9. A method for regulating plant growth, wherein an amount, sufficient for regulating plant growth, of at least one 2-aroylcyclohexanedione of the formula I or of an agriculturally useful salt or ester of I, as claimed in claim 1, is allowed to act on plants or their habitat or on the seed of the plants.

## Revendications

1. 2-aroylcyclohexanediones de formule I 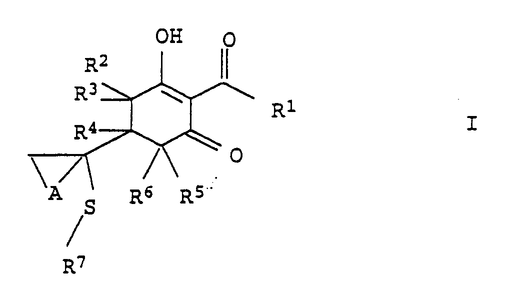 dans laquelle les variables ont la signification suivante:
A méthylène,
R¹ phényle, tandis que le groupe phényle peut être substitué par au plus quatre substituants, choisis à chaque fois dans le groupe consistant en un reste halogéno, cyano, nitro, -N=N-Ph, -S(O)ₘR⁸, (alcoxy en C₁-C₄)carbonyle, -SO₂-N(R⁹)R¹⁰, -N(R⁹)-COR¹⁰, -N(R⁹)-SO₂R¹¹, alkyle en C₁-C₄, alcoxy en C₁-C₄, halogénoalkyle en C₁-C₄, halogénoalcoxy en C₁-C₄, tandis que les quatre restes mentionnés en dernier peuvent quant à eux porter un ou deux des substituants suivants: alcoxy en C₁-C₄, alkylthio en C₁-C₄ et/ou cyano,
Ph représente un reste phényle, qui peut être non-substitué ou peut en option porter un à trois substituants choisis dans le groupe consistant en les restes halogéno, cyano, nitro, -S(O)ₙR²², (alcoxy en C₁-C₄)carbonyle, -SO₂-N(R²³)R²⁴, -N(R²³)-COR²⁴, -N(R²³)-SO₂R²⁵, alkyle en C₁-C₄, alcoxy en C₁-C₄, halogénoalkyle en C₁-C₄ et halogénoalcoxy en C₁-C₄, tandis que les quatre restes mentionnés en dernier peuvent quant à eux porter un ou deux des substituants suivants: alcoxy en C₁-C₄, alkylthio en C₁-C₄ et/ou cyano;
m,n sont à chaque fois 0, 1 ou 2;
R⁸, R²² représentent, indépendamment l'un de l'autre, un reste alkyle en C₁-C₄ ou halogénoalkyle en C₁-C₄, tandis que ces groupes peuvent porter un ou deux restes alcoxy en C₁-C₄, alkylthio en C₁-C₄ et/ou cyano;
R⁹, R¹⁰, R²³, R²⁴ représentent, indépendamment l'un de l'autre, un reste hydrogène, alkyle en C₁-C₄, halogénoalkyle en C₁-C₄, phényle ou phényle portant un à trois restes choisis dans le groupe des restes halogéno, alkyle en C₁-C₄ et alcoxy en C₁-C₄;
R¹¹, R²⁵ représentent, indépendamment l'un de l'autre, un reste alkyle en C₁-C₄ ou halogénoalkyle en C₁-C₄, tandis que ces groupes peuvent porter un ou deux restes cyano, phényle et/ou benzyle;
R², R³, R⁴, R⁵ indépendamment l'un de l'autre, hydrogène ou alkyle en C₁-C₄;
R⁶ hydrogène, alkyle en C₁-C₄ ou (alcoxy en C₁-C₄)carbonyle;
R⁷ alkyle en C₁-C₄;
avec pour condition qu'au moins un substituant du reste phényle soit un reste -S(O)ₘR⁸ avec m=2 et R⁸ un groupe alkyle en C₁-C₄, ainsi que leurs sels utilisables en agriculture, les esters de I avec des acides carboxyliques en C₁-C₁₀ ou des acides minéraux.

2. Procédé pour la préparation de 2-aroylcyclohexanediones de formule I selon la revendication 1, caractérisé par le fait qu'on fait réagir un composé de formule II 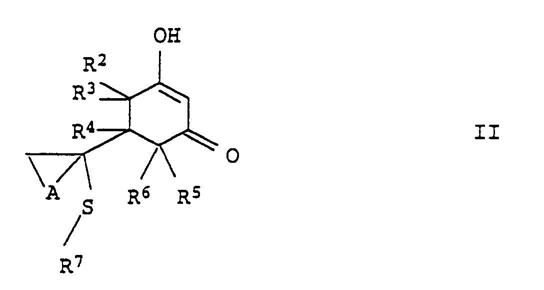 avec un chlorure d'acide de formule III
R¹-CO-L III
dans laquelle L représente un groupe séparable, en présence d'une base dans un solvant inerte,
et on transpose l'énolester de formule IV ainsi obtenu 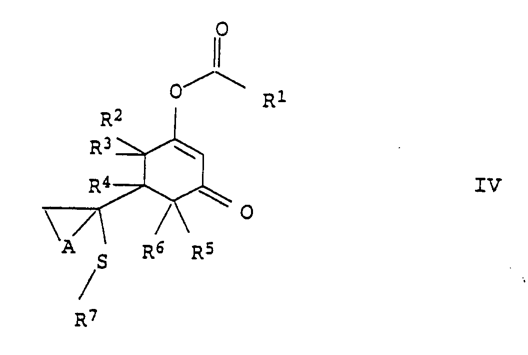 en présence d'une source de cyanure et d'une base dans un solvant inerte.

3. Utilisation des 2-aroylcyclohexanediones I, leurs sels et leurs esters utilisables en agriculture, selon la revendication 1, comme herbicides ou pour la régulation de la croissance des plantes.

4. Agents herbicides, contenant une quantité efficace du point de vue herbicide d'au moins une 2-aroylcyclohexanedione de formule I ou un sel ou un ester de I utilisable en agriculture, selon la revendication 1, et d'au moins un support inerte liquide et/ou solide, ainsi que, en option, au moins un adjuvant.

5. Procédé pour la préparation d'un agent à activité herbicide, caractérisé par le fait qu'on mélange une quantité efficace du point de vue herbicide d'au moins une 2-aroylcyclohexanedione de formule I ou d'un sel ou ester de I, selon la revendication 1, et au moins un support inerte liquide et/ou solide, ainsi que, en option, au moins un adjuvant.

6. Procédé pour la lutte contre la croissance parasite des plantes, caractérisé par le fait qu'on fait agir une quantité efficace du point de vue herbicide d'au moins une 2-aroylcyclohexanedione de formule I ou d'un sel ou un ester utilisable en agriculture de I, selon la revendication 1, sur des plantes, leur biotope ou des semences.

7. Agent pour la régulation de la croissance des plantes, contenant des supports inertes et une quantité suffisante pour la régulation de la croissance des plantes d'une 2-aroylcyclohexanedione de formule I ou d'un sel ou d'un ester de I, selon la revendication 1.

8. Procédé pour la préparation d'agents pour la régulation de la croissance des plantes, caractérisé par le fait qu'on mélange une quantité suffisante pour la régulation de la croissance des plantes d'au moins une 2-aroylcyclohexanedione de formule I ou d'un sel ou ester de I, selon la revendication 1, et au moins un support inerte liquide et/ou solide, ainsi que, en option, au moins un adjuvant.

9. Procédé pour la régulation de la croissance des plantes, caractérisé par le fait qu'on fait agir une quantité suffisante pour la régulation de la croissance des plantes d'au moins une 2-aroylcyclohexanedione de formule I ou d'un sel ou ester utilisable en agriculture de I, selon la revendication 1, sur les plantes, leur biotope ou des semences des plantes.
